# EUROPEAN PATENT APPLICATION

(11) **EP 1 854 785 A1**
(43) Date of publication of application: **14.11.2007**
(21) Application number: 06380112.0
(22) Date of filing: 12.05.2006
(51) Int. Cl.: C07D 211/22, A61K 31/4465, A61P 25/00

(54) **4-[(3-fluorophenoxy)phenylmethyl]piperidine methanesulfonate: uses, process of synthesis and pharmaceutical compositions**

(71) Applicant: FAES FARMA, S.A., 48000 Bilbao Vizcaya (ES)
(72) Inventor: Orjales Venero, Aurelio, 48940 Leioa Vizcaya (ES); Mosquera Pestana, Ramon, 48940 Leioa Vizcaya (ES); Pumar Durán, Ma Carmen, 48940 Leioa Vizcaya (ES); Toledo Avello, Antonio, 48940 Leioa Vizcaya (ES); Canal Mori, Gonzalo, 48940 Leioa Vizcaya (ES); Bordell Martín, Maravillas, 48940 Leioa Vizcaya (ES)
(74) Representative: ABG Patentes, S.L.

(57) **Abstract**

The present patent application is directed to 4-[(3-fluorophenoxy)phenylmethyl] piperidine methanesulfonic acid salt (formula I), its synthesis and use in the manufacture of a medicament for the treatment and/or prevention of a serotonine and/or norepinephrine mediated disease or condition. The present invention is also directed to pharmaceutical compositions comprising the same.

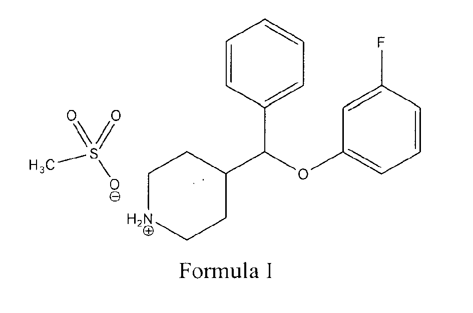

## Description

### FIELD OF THE INVENTION

The present invention refers to a 4-[(3-fluorophenoxy)phenylmethyl]piperidine salt, its use, method of synthesis and compositions comprising the same.

### BACKGROUND OF THE INVENTION

In recent years, selective serotonin (5-HT) reuptake inhibitors (SSRIs) such as fluoxetine, citalopram, sertraline or paroxetine have been used for treating depression and other central nervous system disorders. Potential therapeutic applications of these compounds are treatment of nervous bulimia, alcohol addiction, anxiety, obsessive-compulsive disorders, depression, panic, pain, pre-menstrual syndrome and social phobia, as well as migraine prophylaxis.

On the other hand, dual serotonin and norepinephrine re-uptake inhibitors (SNRls) have been proposed to have a higher efficacy and/or faster onset of action than previously available medicaments in the treatment of depression.

Patent US 6,518,284 B2 and patent application EP1002794 in the name of FAES S.A. describe 4-substituted piperidines having the formula 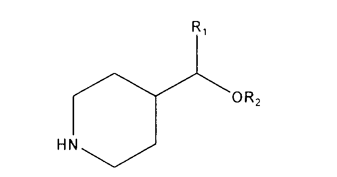 wherein R₁ and R₂ are non-substituted aryl radicals or aryl radicals mono- or polysubstituted with halogen (fluorine, chlorine, bromine, iodine), alkyl, alkoxy, cyano, trifluoromethoxy, trifluoromethyl, benzoyl, phenyl, nitro, amino, aminoalkyl, aminoaryl and carbonylamino, and their pharmaceutically acceptable salts with inorganic acids and organic acids. Said compounds are described as excellent active substances for treating central nervous system disorders such as nervous bulimia, obsessive-compulsive disorders, alcohol addiction, anxiety, panic, pain, pre-menstrual syndrome, social phobia, migraine prophylaxis and, particularly, depression. US 6,518,284 B2 and EP1002794 also describe the synthesis and use of pharmaceutically acceptable salts of said compounds with inorganic acids such as hydrochloric, hydrobromic, nitric, sulphuric and phosphoric; and with organic acids such as acetic, fumaric, tartaric, oxalic, citric, p-toluenesulfonic and methanesulfonic acid. A number of salts of said compounds are disclosed in US 6,518,284 B2 and EP 1002794.

Artaiz, I.; Zazpe, A.; Innerárity, A.; del Olmo, E.; Díaz, A.; Ruiz-Ortega, J.A.; Castro, E.; Pena, R.; Labeaga, L.; Pazos, A. and Orjales, A., Psychopharmacology, 2005, 182(3), 400-413 describes the biochemical, electrophysiological and behavioural assays as antidepressant of (*S*)-4-[(3-fluorophenoxy)phenylmethyl]piperidine which is also known as F-98214-TA.

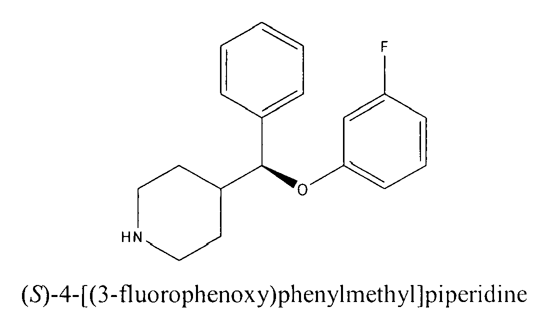

(*S*)-4-[(3-fluorophenoxy)phenylmethyl]piperidine inhibits the uptake of 5-HT and NE into rat brain synaptosomes (IC₅₀ = 1.9 and 11.2 nM, respectively) and decreases the electrical activity of dorsal raphe serotonergic neurones (ED₅₀ = 530.3 microg/kg). In acute behavioural assays in mice, orally administered (*S*)-4-[(3-fluorophenoxy)phenylmethyl]piperidine potentiates the 5-hydroxytryptophan (5-HTP)-induced syndrome [minimal effective dose (MED) = 10 mg/kg], antagonizes the hypothermia induced by a high dose of apomorphine (ED₅₀ = 2 mg/kg) and reduces the immobility in the tail suspension test (MED = 10 mg/kg). (*S*)-4-[(3-fluorophenoxy)phenylmethyl]piperidine also decreases the immobility in the forced swimming test in mice and rats (30 mg/kg, p.o.). Chronic administration of (S)-4-[(3-fluorophenoxy)phenylmethyl]piperidine (14 days, 30 mg kg(-1) day(-1), p. o.) attenuates the hyperactivity induced by olfactory bulbectomy in rats, confirming its antidepressant-like properties. The same dosage regimen significantly increases the social interaction time in rats. Said document also proves that (*S*)-4-[(3-tluorophenoxy)phenylmethyl]piperidine is more potent than fluoxetine, venlafaxine and desipramine.

In view of the above it seems (S)-4-[(3-fluorophenoxy)phenylmethyl]piperidine is an excellent serotonin (5-HT) and norepinephrine (NE) reuptake inhibitor and could therefore be used in the treatment of related diseases and conditions. However, (*S*)-4-[(3-fluorophenoxy)phenylmethyl]piperidine, (*R*)-4-[(3-fluorophenoxy)phenylmethyl] piperidine and their racemic mixtures or its known derivatives, namely their sulphate salt, are not suitable for the preparation of pharmaceutical compositions. As mentioned in US 6,518,284 B2 and EP 1002794, 4-[(3-fluorophenoxy)phenylmethyl]piperidine is an oil, which is insoluble in water and cannot be formulated as a solid composition. 4-[(3-fluorophenoxy)phenylmethyl]piperidine sulfate is a solid with a low melting point (72-76°C). This may be a significant drawback when preparing a pharmaceutical composition as the melting point may be reduced due to the presence of additives or excipients. Additionally, it has been found that 4-[(3-fluorophenoxy)phenylmethyl] piperidine sulfate is obtained with variable amounts of water.

Therefore, there is an existing need of providing a 4-[(3-fluorophenoxy)phenylmethyl]piperidine derivative in a stable and easy to handle form.

### SUMMARY OF THE INVENTION

In the ongoing research effort of the inventors, it has been found that the methanesulfonic acid salt of 4-[(3-fluorophenoxy)phenylmethyl]piperidine has excellent properties for the production of pharmaceutical compositions. Therefore, according to a first aspect, the present invention is directed to 4-[(3-fluorophenoxy)phenylmethyl]piperidine methanesulfonic acid salt of formula I, 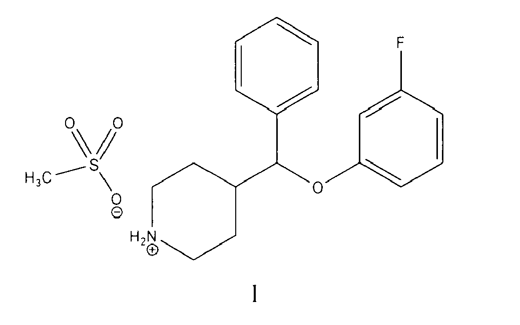 its enantiomers or mixtures thereof, or a prodrug or solvate thereof. Said compound will be referred to as "compound of formula I".

According to a further aspect, the present invention is directed to a process for the synthesis of a compound of formula I, its enantiomers or mixtures thereof, or produrg or solvates thereof.

According to a further aspect, the present invention is directed to a pharmaceutical composition comprising a compound of formula I, its enantiomers or mixtures thereof, or produrg or solvates thereof, and at least one pharmaceutically acceptable carrier.

According to a further aspect, the present invention is directed to a compound of formula I, its enantiomers or mixtures thereof, or produrg or solvates thereof for use as a medicament.

According to a further aspect, the present invention is directed to the use of a compound of formula I, its enantiomers or mixtures thereof, or produrg or solvates thereof in the manufacture of a medicament for the treatment and/or prevention of a serotonine and/or norepinephrine mediated disease or condition.

### DETAILED DESCRIPTION OF THE INVENTION

### Definitions

Any compound that is a prodrug of a compound of formula I is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: carbamates and amides. Examples of well known methods of producing a prodrug of a given acting compound are known to those skilled in the art and can be found e.g. in Krogsgaard-Larsen et al. "Textbook of Drugdesign and Discovery" Taylor & Francis (April 2002). Particularly favoured derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The compound of the invention may be in crystalline form, whether solvated or not, and it is intended that both forms are within the scope of the present invention. Methods of solvation are generally known within the art. Suitable solvates are pharmaceutically acceptable solvates. In a particular embodiment the solvate is a hydrate.

The compounds of formula I its enantiomers or mixtures thereof, or prodrug or solvates thereof are preferably in substantially pure form. By substantially pure form is meant, *inter alia,* having a pharmaceutically acceptable level of purity excluding normal pharmaceutical additives such as carriers, and including no material considered toxic at normal dosage levels. Purity levels for the drug substance are preferably above 50%, more preferably above 70%, most preferably above 90%. In a preferred embodiment it is above 95% of the compound of formula I, or of its solvates or prodrugs.

### Compound of formula I

As mentioned above, a main aspect of the present invention is 4-[(3-fluorophenoxy)phenylmethyl]piperidine methanesulfonic acid salt of formula I, 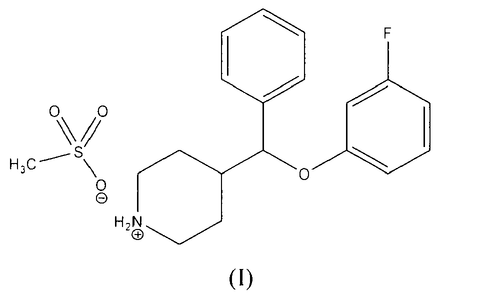 its enantiomers or mixtures thereof, or a prodrug or solvate thereof.

The compound of formula I has a surprisingly high melting point compared to other salts of 4-[(3-fluorophenoxy)phenylmethyl]piperidine. The methanesulfonic acid salt of the mixture of enantiomers of 4-[(3-fluorophenoxy)phenylmethyl]piperidine has a melting point of 158-161°C and the (*S*)-4-[(3-fluorophenoxy)phenylmethyl]piperidine and (*R*)-4-[(3-fluorophenoxy)phenylmethyl]piperidine methanesulfonic acid salts melt at 191-194°C. On the other hand, as an example, the melting point of (*S*)-4-[(3-fluorophenoxy)phenylmethyl]piperidine sulphuric acid salt is highly variable and depends upon the water content, which is difficult to control. (*S*)-4-[(3-fluorophenoxy)phenylmethyl]piperidine hydrochloric acid salt has a melting point of 55-60°C, which is an important drawback when preparing a stable pharmaceutical composition. (*S*)-4-[(3-fluorophenoxy)phenylmethyl]piperidine fumaric acid salt has a melting point of 105-108°C. Although (S)-4-[(3-fluorophenoxy)phenylmethyl]piperidine dibenzoyl-L-tartaric acid salt has a melting point of 205-208°C, it is insoluble in water, thus not suitable for a number of pharmaceutical administration forms such as liquid administration forms. The same is true for the corresponding salts of the other enantiomer of (S)-4-[(3-fluorophenoxy)phenylmethyl]piperidine and for the racemic mixture.

Additionally, the compound of formula I has shown excellent stability properties. The compound of formula I has remained stable for at least 6 months in studies of stability under forced conditions at 50 and 60°C, as well as at 40°C and 75% humidity. This represents more than 2 years of stability under normal conditions.

Further, the compound of formula I is not hygroscopic unlike other salts of (S)-4-[(3-fluorophenoxy)phenylmethyl]piperidine such as the sulphuric or hydrochloric acid salts.

Both enantiomers of the compound of formula I are selective serotonin (5-HT) and norepinephrine (NE) reuptake inhibitors and are therefore suitable for the present invention. Accordingly, a particular embodiment of the present invention is (S)-4-[(3-fluorophenoxy)phenylmethyl]piperidine methanesulfonic acid salt or a prodrug or solvate thereof.

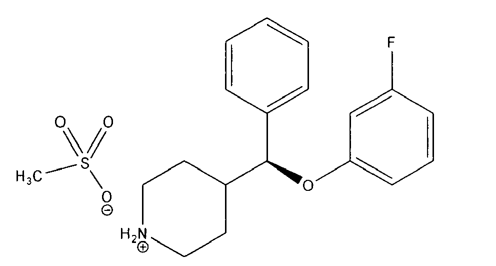

### (S)-4-[(3-fluorophenoxy)phenylmethyl]piperidine methanesulfonic acid salt

According to a particular embodiment, the present invention is directed to (R)-4-[(3-fluorophenoxy)phenylmethyl]piperidine methanesulfonic acid salt or a prodrug or solvate thereof.

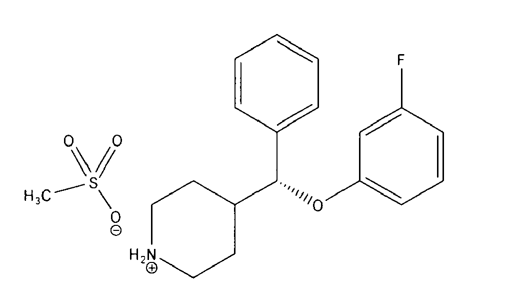

### (R)-4-[(3-fluorophenoxy)phenylmethyl]piperidine methanesulfonic acid salt

According to a further embodiment, the compound of formula I is a mixture of (*S*)-4-[(3-fluorophenoxy)phenylmethyl]piperidine methanesulfonic acid and (*R*)-4-[(3-fluorophenoxy)phenylmethyl]piperidine methanesulfonic acid salt or prodrugs or solvates thereof. Said mixtures may comprise any proportion of both enantiomers. Preferably, the proportions of the R and S isomers in said mixture is comprised between 55:45 and 45:55. More preferably, said mixture is a racemic mixture.

### Synthesis of a compound of formula I

According to a further aspect, the present invention is directed to a process for the synthesis of a compound of formula I, its enantiomers or mixtures thereof, or prodrug or solvates thereof comprising the steps of contacting methanesulfonic acid with 4-[(3-fluorophenoxy)phenylmethyl]piperidine, its enantiomers or mixtures thereof. In order to prepare 4-[(3-fluorophenoxy)phenylmethyl]piperidine, reference is made to US 6,518,284 B2 and EP 1002794, which are hereby incorporated by reference. See column 2, line 64 through column 4, line 20, column 5, lines 64-67 through column 7, lines 1-12; and column 7, lines 13-29 of US 6,518,284. See also the documents cited in US 6,518,284.

Additionally, the compound of formula I can also be synthesized following conventional salt formation procedures. See for example, "Handbook of Pharmaceutical Salts. Properties, Selection and Use". P. Heinrich Stahl, Camille G. Wermouth (Eds.). Wiley-VCH, 2002.

### Pharmaceutical composition comprising a compound of formula I

According to a further aspect, the present invention is directed to a pharmaceutical composition comprising a compound of formula 1 its enantiomers or mixtures thereof, or prodrug or solvates thereof, and at least one pharmaceutically acceptable carrier.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

Further, the term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For its administration to a subject, such as a mammal, e.g., a human, in need of treatment, the pharmaceutical composition of the invention may be administered by any appropriate route (via), such as, oral (e.g., oral, sublingual, etc.), parenteral (e.g., subcutaneous, intramuscular, intravenous, etc.), vaginal, rectal, nasal, topical, ophthalmic, etc.

The carriers and auxiliary substances necessary to obtain the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

### Uses of the compound of formula I and pharmaceutical compositions

The compound of formula I, its enantiomers or mixtures thereof, or prodrug or solvates thereof, may be used as active ingredient of medicaments. Thus, according to a further aspect, the present invention relates to a compound of formula I, its enantiomers or mixtures thereof, or a prodrug or solvate thereof, for use as a medicament.

Concretely, the compound of formula I is a dual serotonin (5-HT) and norepinephrine (NE) reuptake inhibitor (SNRI). Thus, according to a further aspect, the present invention is directed to the use of a compound of formula I, its enantiomers or mixtures thereof, or a prodrug or solvate thereof, in the manufacture of a medicament for the treatment and/or prevention of a serotonine and/or norepinephrine mediated disease or condition, preferably, a central nervous system disorder.

According to a preferred embodiment, said central nervous system disorder is selected from the group consisting of nervous bulimia, alcohol addiction, anxiety, obsessive-compulsive disorders, panic, pain, pre-menstrual syndrome, social phobia, depression and migraine prophylaxis. According to a further preferred embodiment, said central nervous system disorder is depression.

According to a further aspect, the present invention is directed to a method for treating a serotonine and/or norepinephrine mediated disease or condition, preferably, a central nervous system disorder, more preferably, depression, in a human in need of such treatment by administering a therapeutically effective amount of a compound of formula I, its enantiomers or mixtures thereof, or a prodrug or solvate thereof.

In the sense used in this description, the expression "therapeutically effective amount" refers to the quantity of active ingredient calculated to produce the desired effect and will generally be determined, among other reasons, by the own features of the active ingredient used and the therapeutic effect to be obtained. In a particular embodiment, the dose of active ingredient administered to a subject in need of treatment for the treatment and/or prophylaxis of the above mentioned conditions is within the range of 10⁻⁴ to 10³ mg/kg of body weight, preferably 10⁻¹ to 10² mg/kg of body weight.

The following examples can be used to illustrate the invention and must not be considered to be limiting of the scope thereof.

### EXAMPLES

### Example 1

### Synthesis of (±)-4-[(3-fluorophenoxy)phenylmethyl]peperidine

A NaH (0.40 g, 60% mineral oil) suspension in 6 ml DMSO was treated with a solution of (±)-4-(hydroxyphenylmethyl)piperidine-1-carboxylic acid tert-butyl ester (2.55 g, 8.75 mmol) in 6 ml of DMSO. Potassium benzoate (1.35 g, 8.43 mmol) and 1,3-difluorobenzene (1.05 ml, 10.6 mmol) were added, and the reaction mixture was heated to 85°C until the starting substance disappeared. It was then treated with saturated aqueous NaCl and water solution, and extracted with diethyl ether. The organic phase evaporation residue was treated with methanol (30 ml) and 10% aqueous HCl solution (30 ml) and refluxed for an hour. The usual reaction working process yielded 2.16 g of free base as an amber oil (88% yield).

¹H NMR (200 MHz, CDCl₃): δ = 7.37-7.03 (m, 6H), 6.65-6.46 (m, 3H), 4.78 (d, *J*= 6.4 Hz, 1H), 3.08 (m, 2H), 2.55 (m, 2H), 1.98-1.81 (m, 2H), 1.43-1.22 (m, 3H). ¹³C NMR (50 MHz, CDCl₃): δ = 163.3 (d, *J* = 233.1 Hz), 159.7 (d, *J* = 10.7 Hz), 139.4, 129.8 (d, *J* = 9.8 Hz), 128.3, 127.6, 126.6, 111.5 (d, *J* = 3.0 Hz), 107.3 (d, *J* = 21.0 Hz), 103.5 (d, *J* = 23.4 Hz), 84.6, 46.4, 46.4, 43.4, 29.5, 29.2

### Example 2

### Synthesis of (±)-4-[(3-fluorophenoxy)phenylmethyl]piperidine methanesulfonic acid salt

To 1.06 g (3.71 mmol) of (±)-4-[(3-fluorophenoxy)phenylmethyl]piperidine dissolved in 10 ml of 2-butanone, 0.22 ml (3.34 mmol) of methanesulfonic acid were dropped. The solvent was evaporated under vacuum and the white solid recrystallized from 5.2 ml ofn-butanol yielding 0.75 g (mp 159.0-160.6°C).

### Example 3

### Resolution of (+)-4-[(3-fluorophenoxy)phenylmethyl]piperidine

4.45 g of (-)-O,O'-dibenzoyl-L-tartaric acid were added over 7.1 g (25 mmol) of (±)-4-[(3-fluorophenoxy)phenylmethyl]piperidine dissolved in 175 ml of ethanol (96%). A white solid was obtained (mp 212°C) which was treated with 5% aqueous NaOH solution and extracted with chloroform, yielding the (S)-enantiomer (96% e.e., mp 59-62 °C, [α]₅₄₆ = -11.4, c = 0.576, CHCl₃).

The filtrated liquids were treated with aqueous NaOH (5%) solution and chloroform. The organic layer was separated, dried and concentrated. The product obtained, dissolved in ethanol was treated with (+)-2,3-dibenzoyl-D-tartaric acid using the preceding process. A white solid was obtained (mp 208°C) which was treated with aqueous NaOH (5%) solution and extracted with chloroform, yielding the (R)-enatiomer (98% e.e., mp 59-62°C, [α]₅₄₆ = +11.4, c = 0.618, CHCl₃).

### Example 4

### Synthesis of (S)-phenyl(piperidin-4-yl)methanol (1S)-(+)10-camphorsulfonic acid salt

To a solution of (-)-DIPCl (315 mL, 0.57 mol, 63.6 % in heptane) in THF anhydrous (1 1), 4-(benzoyl)piperidine-1-carboxylic acid tert-butyl ester (75.0 g, 0.26 mol) was added at room temperature. After 21 hours acetaldehyde (55 mL) was added, stirring continued for 3 h at room temperature and then 25 % aq NaOH added and the mixture stirred for 45 min. The organic phase was separated, washed with brine, the solvent removed under reduced pressure and the residue treated with dichloromethane. The aqueous phase was treated with 30 % aqueous NaOH, extracted with dichloromethane, dried, filtered and concentrated to give a brown oil (56 g) wich was dissolved in THF (300 mL), treated with (1S)-(+)-camphorsulphonic acid (51.9 g) and heated until 85 °C. After 20 h at room temperature a white solid was filtered (mp 148.1-150.9 °C, yield: 55.3 %, 99.9 % ee)

### Example 5

### Asymmetric synthesis of (S)-4-[(3-fluorophenoxy)phenylinethyl]piperidine

To a solution the (S)-phenyl(piperidin-4-yl)methanol (1S)-(+)-10-camphorsulfonic acid salt (15g, 35.4 mmol), prepared in example 4, in DMSO (100 ml), 10.04 g of potassium tert-butoxide were added at room temperature. The mixture was stirred for 2 h and then 1,3-difluorobenzene (4.2 ml) was dropped maintaining the temperature below 25°C. After 16 h, water (500 ml), saturated solution of NaCl (100 ml) and dichloromethane (500 ml) were added and the layers shaked. The organic one was separated, washed with water, dried and the solvent removed. A clear oil was obtained, 7.85 g (e.e. 99.66%).

### Example 6

### Asymmetric synthesis of (R)-4-[(3-fluorophenoxy)phenylmethyl]piperidine

A solution of (R)-phenyl(piperidin-4-yl)methanol (1.5 g, 7.84 mmol) in DMSO (18 ml) was dropped on a suspension of NaH (0.52 g, 10.9 mmol) in DMSO (18.5 ml) at room temperature. Potassium benzoate (1.3 g, 8.05 mmol) and 1,3-difluorobenzene (1.14 g, 10.04 mmol) were added and the mixture stirred for 20 h at 40°C. Then, it was poured over water (29 ml) and aqueous NaCl saturated solution (37 ml), and extracted three times with ethyl ether. The joined organic layers were dried and the solvent removed. The residue was treated with hexane and 10 % aqueous HCl and the aqueous phase extracted with chloroform. The organic phase was washed with 10 % aqueous NaOH, dried (anhydrous Na₂SO₄), filtered and concentrated to give an oil which was dissolved in ethanol (120 mL) and treated with dibenzoyl-D-tartaric acid (1.4 g, 3.92 mmol, 0.5 eq). A precipitated was observed, stirred over 15 min and filtered. The solid was treated with 10 % aqueous NaOH (30 mL), extracted with CH₂Cl₂ and the organic phase dried over anhydrous Na₂SO₄, filtered and the solvent removed to give a pale yellow oil (0.6 g" yield: 27 %, 97.6 % ee).

### Example 7

### Synthesis of (S)-4-[(3-fluorophenoxy)phenylmethyl]piperidine methanesulfonic acid salt

Methanesulfonic acid (1.3 ml, 20.45 mmol) was added to a solution of (S)-4-[(3-fluorophenoxy)phenylmethyl]piperidine (6.14 g, 21.53 mmol) in 4-methyl-2-pentanone (50 ml) at 95°C. The white crystals were filtered and dried (6.47 g, 82.8% yield, 99.80% enantiomeric excess).

### Example 8

### Synthesis of (R)-4-[(3-fluorophenoxy)phenylmethyl]piperidine methanesulfonic acid salt

(R)-4-[(3-fluorophenoxy)phenylmethyl]piperidine (5.5 g, 18.22 mmol) was dissolved in isopropanol (18.2 ml) at 65°C and methanesulfonic acid (1.12 ml, 17.31 mmol) added. The white crystals were filtered an dried (5.7 g, 77.5% yield, 99.76% enantiomeric excess).

### Example 9

### Serotonin (5-HT) reuptake inhibitory activity of (S)-4-[(3-fluorophenoxy)phenylmethyl]piperidine methanesulfonic acid salt

Adult male Wistar rats weighing 220-280 g were used. Animals were sacrificed by guillotine decapitation and the whole brain quickly removed. Frontal cortex tissue was placed in ice-cold 0.32 M sucrose (1:10 w/v) and homogenized with a motor-driven teflon pestle Potter-S homogenizer (12 strokes, 800 rpm). The homogenates were centrifuged at 1,500 g for 10 min at 4 °C. The pellet (P1) was discarded and the supernatant was centrifuged at 18,000 *g* for 10 min at 4°C. The final pellet (P2) was suspended in Krebs-bicarbonate physiological buffer (composition: 120.8 mM NaCl, 5.9 mM KCl, 2.2 mM CaCl₂, 1.2 mM MgCl₂·6H₂O, 1.2 mM NaH₂PO₄, 15.5 mM NaHCO₃ and 11.5 mM α-D-glucose solution) (pH 7.4) gassed under 95% O₂ and 5% CO₂ for 10 min at room temperature.

The synaptosomal suspensions were incubated in a shaking water bath at 37°C for 15 min. Aliquots (150 µl) were then added to tubes containing 275 µl of Krebs-physiological buffer and 50 µl buffer (total uptake) or 50 µl of drug solution (at concentrations ranging from 10⁻¹⁰ to 10⁻⁴ M) or 50 µl 10 µM fluoxetine (non-specific uptake). Uptake was initiated by the addition of 25 µl [³H]-5-HT (20 nM) followed by incubation for 2 min at 37°C in shaking water bath. Process was stopped and membranes were filtered through Whatman GF/B filters presoaked in uptake buffer containing 0.05-0.1% polyethyleneimine, using a Brandel M-24R cell harvester Filters were immediately rinsed three times with 4 ml ice-cold solution, dried and immersed into polyethylene vials containing 5 ml of Ecoscint-H scintillation cocktail The filter-retained radioactivity was determined by scintillation counting. Data were analyzed by nonlinear regression models and the concentration-effect curve was constructed (GraphPad Prism, version 2.0); to determine IC₅₀ values. For each assayed drug IC₅₀ mean value was obtained from a minimum of three independent experiments using 6-8 drug concentrations.

IC₅₀ values were calculated for (*S*)-4-[(3-fluorophenoxy)phenylmethyl] piperidine methanesulfonic acid salt (F-98214-TA3) and the compounds used as reference. The values are shown in Table I:

**Table I**

| Drug | IC₅₀ (nM) |
|---|---|
| | mean ± sem |
| F98214-TA3 | 1.91 ±0.37 |
| Fluoxetine | 59.9 ± 14.9 |
| Venlafaxine | 116.7 ± 19.0 |
| Desipramine | >1000 |

### Example 10

### Norepinephrine (5-HT) reuptake inhibitory activity of (S)-4-[(3-fluorophenoxy) phenylmethyl]piperidine methanesulfonic acid salt

Adult male Wistar rats weighing 220-280 g were used. Animals were sacrificed by guillotine decapitation and the whole brain quickly removed. Frontal cortex tissue was placed in ice-cold 0.32 M sucrose (1:10 W/V) and homogenized with a motor-driven teflon pestle Potter-S homogenizer (12 strokes, 800 rpm). The homogenates were centrifuged at 1,500 g for 10 min at 4 °C. The pellet (P1) was discarded and the supernatant was centrifuged at 18,000 g for 10 min at 4°C. The final pellet (P2) was suspended in Krebs-bicarbonate physiological buffer (composition : 120.8 mM NaCl, 5.9 mM KCl, 2.2 mM CaCl₂, 1.2 mM MgCl₂·6H₂O, 1.2 mM NaH₂PO₄, 15.5 mM NaHCO₃ and 11.5 mM α-D-glucose solution) (pH 7.4) gassed under 95% O₂ and 5% CO2 for 10 min at room temperature.

The synaptosomal suspensions were incubated in a shaking water bath at 37°C for 15 min. Aliquots (150 µl equivalent to 2.5 mg wett weight tissue) were then added to tubes containing 275 µl of Krebs-physiological buffer and 50 µl buffer (total uptake) or 50 µl of drug solution (at concentrations ranging from 10⁻¹⁰ to 10⁻⁴ M) or 50 µl 10 µM nisoxetine (non-specific uptake). Uptake was initiated by the addition of 25 µl [³H]-NA (10 nM) followed by incubation for 5 min at 37°C in shaking water bath. Process was stopped and membranes were filtered through Whatman GF/B filters presoaked in uptake buffer containing 0.05-0.1 % polyethyleneimine, using a Brandel M-24R cell harvester Filters were immediately rinsed three times with 4 ml ice-cold solution, dried and immersed into polyethylene vials containing 5 ml of Ecoscint-H scintillation cocktail The filter-retained radioactivity was determined by scintillation counting. Data were analyzed by nonlinear regression models and the concentration-effect curve was constructed (GraphPad Prism, version 2.0); to determine IC₅₀ values. For each assayed drug IC₅₀ mean value was obtained from a minimum of three independent experiments using 6-8 drug concentrations.

(*S*)-4-[(3-fluorophenoxy)phenylmethyl]piperidine methanesulfonic acid salt showed nanomolar potency (IC₅₀= 11.15± 4.88 nM) as an inhibitor of noradrenaline uptake. This potency is similar to those showed by duloxetine and reboxetine.

## Claims

1. 4-[(3-fluorophenoxy)phenylmethyl]piperidine methanesulfonic acid salt of formula I, 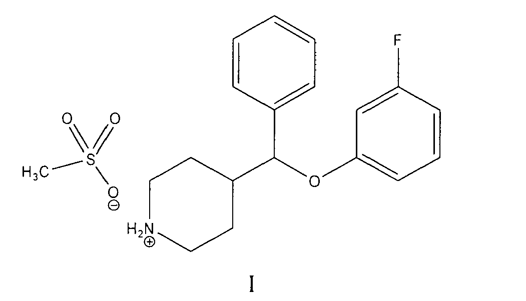 its enantiomers or mixtures thereof, or a prodrug or solvate thereof.

2. (*S*)-4-[(3-fluorophenoxy)phenylmethyl]piperidine methanesulfonic acid salt or a prodrug or a solvate thereof.

3. (*R*)-4-[(3-fluorophenoxy)phenylmethyl]piperidine methanesulfonic acid salt or a prodrug or a solvate thereof.

4. Compound according to claim 1 as a mixture of (*S*)-4-[(3-fluorophenoxy)phenylmethyl]piperidine methanesulfonic acid and (*R*)-4-[(3-fluorophenoxy)phenylmethyl]piperidine methanesulfonic acid salt or prodrugs or solvates thereof.

5. A process for the synthesis of a compound as defined in any of claims 1 to 4 comprising the steps of contacting methanesuofonic acid with 4-[(3-fluorophenoxy)phenylmethyl]piperidine, its enantiomers or mixtures thereof.

6. A pharmaceutical composition comprising a compound as defined in any of claims 1 to 4, and at least one pharmaceutically acceptable carrier.

7. A compound as defined in any of claims 1-4 for use as a medicament.

8. Use of a compound as defined in any of claims 1-4 in the manufacture of a medicament for the treatment and/or prevention of a serotonine and/or norepinephrine mediated disease or condition.

9. Use according to claim 8 wherein said serotonine and/or norepinephrine mediated disease or condition is a central nervous system disorder.

10. Use according to claim 9 wherein said central nervous system disorder is selected from the group consisting of nervous bulimia, alcohol addiction, anxiety, obsessive-compulsive disorders, panic, pain, pre-menstrual syndrome, social phobia, depression and migraine prophylaxis.

11. Use according to claim 10 wherein said central nervous system disorder is depression.
